# EUROPEAN PATENT SPECIFICATION

(11) **EP 1 032 324 B1**
(45) Date of publication and mention of the grant of the patent: **06.10.2004**
(21) Application number: 98960947.4
(22) Date of filing: 13.11.1998
(51) Int. Cl.: A61C 15/04, A61K 7/16

(54) **DENTAL FLOSS**
ZAHNSEIDE
FIL DENTAIRE

(30) Priority: 17.11.1997 BR 9705386
(43) Date of publication of application: 06.09.2000
(73) Proprietor: Johnson & Johnson Industrial Ltda., Sao José dos Campos, SP (BR)
(72) Inventor: NETTO, Emilson, Ismael Apartamento 41, CEP-12300-000 Jacarei, SP (BR); SIMIONATO, Luiz, Bellino, CEP-12243-700 Sao José dos Campos, SP (BR); MELNIK, Joseph, David, CEP-01411-011 Sao Paulo, SP (BR)
(74) Representative: Mercer, Christopher Paul
(86) International application number: PCT/BR1998/000106
(87) International publication number: WO 1999/025269

(56) References cited:
- EP-A- 0 637 446
- WO-A-93/02633
- WO-A-95/34275
- US-A- 5 455 024

## Description

### Field of the Invention

The present invention refers to a dental floss and, more particularly, to strands and tapes for interdental cleaning, having improved characteristics of lubricity and of power for removing food remnants which are entrapped in the interproximal dental regions.

### Background of the Invention

A well known embodiment for dental strands and tapes (for simplification hereinafter named dental flosses) consists of a braid of filaments from cotton, silk or several polymeric materials, which is impregnated in a heated bath with an additive composition containing a wax as a carrier, and one or more additives among germicides, therapeutic agents, flavorants, sweeteners and abrasives, among others. The cooled additive composition forms a coating on the dental floss in which, besides aggregating the several additives, the wax has the functions of aggregating the braided filaments and of acting as a lubrifying agent, facilitating the introduction of the strand in the interproximal dental regions, as well as its sliding during application.

However, in spite of their good lubricity characteristics, due to the braided, thus cohesive disposition of their filaments, these dental flosses are not provided with anchor points which allow the holding and consequent removal of food particles with its use. Such filamentary compaction, since it allows only the superficial incorporation of the coating, it also limits this type of dental floss because of the small antiseptic and therapeutic load it may incorporate.

To overcome these limitations, the so-called texturized dental flosses have been developed, in which filaments are unbraided and then impregnated with the additive composition. Besides having a greater contact surface with the interproximal dental surfaces, as well as a good capability for removing food particles which are entrapped in the interstices of the loose filaments, the texturized dental flosses have the additional advantage of incorporating substantially larger amounts of additive composition in the interfilamentary spaces, providing a final product with greater antiseptic and therapeutic power.

However, it has been observed that a wax-based additive composition, such as that described above, when applied to a texturized dental floss, upon the winding thereof at the feeding means of the dental floss packaging station, suffers an exudation which makes the strand surface sticky, impairing the next packaging step and also making the strand unpleasant to be handled by the user. It has been noted that this problem is due to the fact that the wax stiffens externally, preventing the drying of the interfilamentar portion of the coating, the remaining humidity being "squeezed out" of the strand due to the winding tension of the strand at the feeding means.

In order to overcome this problem, there were developed texturized strands, in which the wax, as a carrier, was eliminated. An example of this solution is the dental floss object of patent US 5,098,711, assigned to Hill et al. at 03.24.92, which mentions in its additive composition just a small amount of polyethylene wax, the function of which is to deposit in the interproximal dental regions during the application of the strand, forming a protective coating during a period of time after the cleaning.

This texturized strand, on the other hand, due to the lack of lubrication provided by the wax becomes rough in use, besides transmitting to the user the sensation that it is fraying. Due to these unpleasant sensations, a part of the consumer market rejects this type of dental floss.

EP-A-0637446 discloses a dental floss provided with chemotheraphy agents, characterized in that said floss including a wax that is emulsible upon contact with saliva, said emulsible wax including an insoluble wax, which may be beeswax, and at least one surfactant capable of emulsifying said insoluble wax upon contact with saliva.

WO 93 02633 discloses a flattened dental cleaning article and a process for making it. A multi-filament thread is coated with a first liquid or semi-solid wax composition having a high melting temperature and then, after flattening the cooled thread is coated with a lower melting liquid or semi-solid wax composition. The lower melting wax composition contains volatile flavouring oil and, optionally, xylitol.

WO 9534275 discloses an oral hygiene composition comprising a combination of a particulate cellulose cleaning/polishing agent and an agent to provide an enhanced cleaning/polishing action selected from one or more of: an alkali metal bicarbonate, an alkali metal orthophosphate, an alkali metal citrate and any compatible mixtures thereof.

US-A-5455024 discloses to dentifrices such as toothpastes, tooth gels, or tooth powders to which are added zinc oxide particles, preferably agglomerated. The dentifrices contain sodium bicarbonate and other conventional ingredients such as thickeners, anti-calculus agents such as a pyrophosphate salts, anti-caries agents such as sodium fluoride, flavourings, sweetening agents, and/or secondary abrasives.

### Disclosure of the Invention

In one aspect the present invention provides a texturized dental floss, which combines the characteristics of high power for removing food remnants entrapped in the interproximal dental regions with a high lubricity, allowing the user to have an efficient and pleasant application.

It is also an object of in another aspect the present invention provides a dental floss as described above, that although coated with a wax based additive composition, is of easy manufacturing and packaging, without presenting exudations derived from the humidity retained in the coating, upon drying. In still another aspect the present invention provides a dental floss as described above, which has a substantially enlarged antiseptic and/or therapeutic load.

In a further aspect the present invention provides a dental floss as described above, which provides a controlled release of the antiseptic and/or therapeutic load of its coating.

According to the present invention there is provided a dental floss as defined in the appendant claims.

The water emulsionable carrier, of the dental floss may carry one or more active agents selected from the group consisting of germicides, therapeutic agents, flavorants, sweeteners, abrasives and others, at least one sweetener being in aqueous solution.

In practical terms, the dental floss of the invention incorporates the advantages of both the braided and the texturized dental flosses. Since it utilizes wax in its coating composition, the strand of the invention has great lubricity, making pleasant its application by the user and, although being texturized, it has reduced transversal dimensions, due to the aggregation of its filaments by the wax, facilitating the insertion of said dental floss in the interproximal dental regions.

Furthermore, due to the texturized disposition of its filaments, the dental floss of the invention also incorporates the coating composition in its interfilamentary regions, resulting in a substantially increased load of said composition and therefore of the antiseptic and/or therapeutic agents.

Additionally, since the wax is emulsionable in water, the filaments progressively separate from each other and, with the consequent transversal dimensional increase of the strand, the latter will clean more efficiently the interproximal dental surfaces, as well as remove the food remnants which are entrapped within the filaments. Furthermore, the coating composition of said dental floss releases its load of active substances as the wax is progressively, thus controllably, dissolved in the saliva, extending the effect of its antiseptic and/or theraupetic load, as well as the pleasant flavor provided by the flavoring and sweetening agents.

Finally, the dental floss of the invention presents an extremely soft texture.

### Detailed Description of the Preferred Embodiment

The present invention refers to a dental floss, which is to be used for hygiene and/or interdental therapy and which is composed of multiple filaments, which are made from one or more materials selected among cotton, silk, nylon, PTFE and other polymeric materials.

According to the invention, said filaments are opened through a texturizing treatment, preferably by heat, of a filamentar braid, or basic strand, which is commercially available, being obtained a texturized strand which is next impregnated, by passing through a heated bath, with a coating composition, resulting in the coated dental floss which is wound in a feeding means, wherefrom it is discharged for packaging.

The coating composition of the dental floss comprises an emulsion of a water emulsionable carrier, carrying one or more active agents selected among germicides, therapeutic agents, flavorants, sweeteners, abrasives and others, such as antioxidants. The water emulsionable carrier includes a waxy material selected from the group consisting of animal waxes, mineral waxes, vegetable waxes, synthetic waxes and mixtures of two or more thereof; and an emulsifier selected among glyceryl monoestearate, ethoxilated glyceryl monoestearate, sorbitane monoestearate and mixtures thereof.

In the preferred embodiment of the invention, the water emulsionable carrier includes: 4 to 10% by weight, based on the total weight of the coating composition, of a mineral wax, such as a microcrystalline wax, and from 25 to 40% by weight, based on the total weight of the coating composition, of a refined bee wax; and the emulsifier includes from 3 to 10% by weight, based on the total weight of the coating composition, of glyceryl monoestearate, 22 to 30% by weight, based on the total weight of the coating composition, of ethoxilated glyceryl monoestearate, and from 23 to 35% by weight, based on the total weight of the coating composition, of sorbitane monoestearate.

In the composition of the water emulsionable carrier, the mixture of the microcrystalline and bee waxes has the primary functions of lubricating the strand, aggregating its filaments and carrying the active agents, as described below. Furthermore, the microcrystalline and bee waxes have the secundary functions of bodyfying the coating and providing the characteristic of softness in the contact point of the dental floss with its application area, respectively.

Accordingly, in the case of the emulsifiers, the glyceryl monoestearate and the sorbitane monoestearate share secundary functions of providing consistency and softness to the coating composition.

In a basic composition, designed exclusively to interdental hygiene, one or more flavorants, a sweetener and an abrasive are spread in the water emulsionable carrier as active agents.

In a coating composition, such as that described above, it has been noted that a water content above approximately 4% by weight, based on the total weight of said composition, causes the exudation thereof, this being also promoted by the flavor agent when used as oil.

To overcome the exudation problem, the coating composition utilizes from 10 to 25% by weight, based on its total weight, of one or more flavorants, which are microencapsulated in special arabic gum or modified starch, for example, as commercially available from the flavorant suppliers.

All known sweeteners are solid and water soluble, therefore the water contained in the coating composition originates from the dissolution of the sweetener.

Therefore, in order to reduce the water content of the coating composition of the dental floss of the invention, it is utilized a powerful sweetener, for example those at least 600 times sweeters than sugar, such as sucralose, alitame, etc., and preferably, from about 0.5 to about 3.0% by weight of a 25% sucralose solution, based on the total weight of the composition.

To help in the removal of build-ups, preventing the formation of tartar in the interproximal dental regions, the coating composition of the proposed dental floss further includes a weak abrasive, preferably silica, in an amount from 2 to 15% by weight, based on the total weight of said composition.

Besides the abrasive effect, the silica improves the dental floss surface to be held by the user, being also able to absorb any minimal amount of liquid (water and/or oil) that exudes from the strand coating.

The dental floss of the invention is extremely soft and since it has its filaments aggregated by the coating wax, it is initially slim and therefore easy to insert in the interproximal dental regions.

As the dental floss is applied, the coating is progressively released and deposited on the application regions and, simultaneously, the filaments progressively separate and occupy larger spaces of these regions, starting to capture food remnants in the interstices of the filaments and to friction the deposited coating against the interproximal dental regions, removing occasional build-ups and thus avoiding the formation of tartar, etc., by action of silica.

During the whole period of dental floss application, as described above, and during a period after that, said wax is progressively emulsified by the saliva and consequently releases its active composition, providing the user with a lasting and pleasant sensation of cleanness.

As mentioned in the beggining of the description of the invention, the latter was illustrated through the basic embodiment of a dental floss designed only for interdental hygiene. It should be understood, however, that to this basic formulation may be added germicides, pharmaceutical compounds or any desired specific active agents to provide the dental floss with additional antiseptic and/or therapeutic characteristics.

## Claims

1. Dental floss, of the type comprising a strand consisting of multiple filaments which are made from one or more materials selected from the group consisting of cotton, silk and polymeric materials; and a coating composition, impregnating said strand and comprising a water emulsionable carrier, carrying one or more active agents selected from the group consisting of germicides, therapeutic agents, flavorants, sweeteners, abrasives and others, at least one sweetener being in aqueous solution, said water emulsionable carrier comprising a waxy material selected from the group consisting of animal waxes, mineral waxes, vegetable waxes, synthetic waxes and mixtures of two or more of these waxes, and an emulsifyer, in order to make said coating composition homogeneous and soluble in saliva, wherein the sweetener aqueous solution has a sufficiently low amount of water to avoid the exudation of the impregnated strand.

2. Dental floss, according to claim 1, **characterized in that** the coating composition further comprises, as a flavorant, from 10 to 25% by weight of at least one microencapsulated flavorant, based on the total weight of said coating composition.

3. Dental floss, according to claim 1, **characterized in that** the coating composition comprises, as a sweetening agent, sweeteners which are at least 600 times sweeter than sugar.

4. Dental floss, according to claim 1, **characterized in that** the coating composition further comprises, as a sweetening agent, from 0.5 to 3% by weight of an aqueous solution at 50% of sucralose.

5. Dental floss, according to claim 1, **characterized in that** the coating composition further comprises, as an abrasive agent, from 2 to 15% by weight of silica, based on the total weight of said coating composition.

6. Dental floss, according to claim 1, **characterized in that** the total content of water of said coating composition is no more than 4% by weight of said composition.

7. Dental floss, according to claim 1, **characterized in that** the strand is texturized by heat.

## Patentansprüche

1. Zahnseide des Typs, die einen Strang, der aus mehreren Fäden, die aus einem oder mehreren Materialien ausgewählt aus der Gruppe bestehend aus Baumwolle, Seide und polymeren Materialien besteht; und eine Beschichtungszusammensetzung umfaßt, die den Strang imprägniert und einen wasseremulgierbaren Träger umfaßt, der ein oder mehrere aktive Wirkstoffen ausgewählt aus der Gruppe bestehend aus Germiziden, therapeutischen Mitteln, Geschmacksstoffen, Süßstoffen, Schleifmitteln und andere trägt, wobei mindestens ein Süßstoff in wäßriger Lösung ist, der wasseremulgierbare Träger ein wachsartiges Material ausgewählt aus der Gruppe bestehend aus Tierwachsen, Mineralwachsen, Pflanzenwachsen, synthetischen Wachsen und Gemischen von zwei oder mehreren dieser Wachse und einen Emulgator umfaßt, um die Beschichtungszusammensetzung homogen und im Speichel löslich zu machen, wobei die wäßrige Süßstofflösung einen ausreichend niedrigen Wassergehalt aufweist, um die Ausscheidung aus dem imprägnierten Strang zu vermeiden.

2. Zahnseide gemäß Anspruch 1, dadurch charakterisiert, daß die Beschichtungszusammensetzung des weiteren als Geschmacksstoff beruhend auf dem Gesamtgewicht der Beschichtungszusammensetzung von 10 bis 25 Gew.% mindestens eines mikroeingekapselten Geschmacksstoffs umfaßt.

3. Zahnseide gemäß Anspruch 1, dadurch charakterisiert, daß die Beschichtungszusammensetzung als einen Süßstoff Süßstoffe umfaßt, die mindestens 600 mal süßer als Zucker sind.

4. Zahnseide gemäß Anspruch 1, dadurch charakterisiert, daß die Beschichtungszusammensetzung des weiteren als einen Süßstoff von 0,5 bis 3 Gew.% einer wäßrigen Lösung mit 50% Sucralose umfaßt.

5. Zahnseide gemäß Anspruch 1, dadurch charakterisiert, daß die Beschichtungszusammensetzung des weiteren als ein Schleifmittel beruhend auf dem Gesamtgewicht der Beschichtungszusammensetzung von 2 bis 15 Gew.% Silica umfaßt.

6. Zahnseide gemäß Anspruch 1, dadurch charakterisiert, daß der Gesamtwassergehalt der Beschichtungszusammensetzung nicht mehr als 4 Gew.% der Zusammensetzung ausmacht.

7. Zahnseide gemäß Anspruch 1, dadurch charakterisiert, daß der Strang durch Hitze texturiert wird.

## Revendications

1. Fil dentaire, du type comprenant un brin constitué de filaments multiples qui sont faits d'un ou plusieurs matériaux choisis dans le groupe constitué du coton, de la soie et de matériaux polymères ; et une composition de revêtement, imprégnant ledit brin et comprenant un support pouvant être émulsionné dans l'eau, portant un ou plusieurs agents actifs choisis dans le groupe constitué de germicides, agents thérapeutiques, essences, édulcorants, abrasifs et autres, au moins un édulcorant étant en solution aqueuse, ledit support pouvant être émulsionné dans l'eau comprenant un matériau cireux choisi dans le groupe constitué des cires animales, cires minérales, cires végétale, cires synthétiques et mélange de deux de ces cires ou plus, et un émulsifiant, pour rendre ladite composition de revêtement homogène et soluble dans la salive, dans lequel la solution aqueuse d'édulcorant possède une quantité d'eau suffisamment faible pour éviter l'exsudation du brin imprégné.

2. Fil dentaire selon la revendication 1, **caractérisé en ce que** la composition de revêtement comprend en outre, comme essence, de 10 à 25% en poids d'au moins une essence microencapsulée, sur la base du poids total de ladite composition de revêtement.

3. Fil dentaire selon la revendication 1, **caractérisé en ce que** la composition de revêtement comprend, comme agent édulcorant, des édulcorants qui sont au moins 600 fois plus sucrés que le sucre.

4. Fil dentaire selon la revendication 1, **caractérisé en ce que** la composition de revêtement comprend, en outre, comme agent édulcorant, de 0,5 à 3% en poids d'une solution aqueuse à 50% de sucralose.

5. Fil dentaire selon la revendication 1, **caractérisé en ce que** la composition de revêtement comprend, en outre, comme agent abrasif, de 2 à 15% en poids de silice, sur la base du poids total de ladite composition de revêtement.

6. Fil dentaire selon la revendication 1, **caractérisé en ce que** le contenu total en eau de ladite composition de revêtement n'est pas supérieur à 4% en poids de ladite composition.

7. Fil dentaire selon la revendication 1, **caractérisé en ce que** le brin est texturé par de la chaleur.
